(19) Europäisches Patentamt
European Patent Office
Office européen des brevets

(11) EP 1 002 522 A1

(12) EUROPÄISCHE PATENTANMELDUNG

(43) Veröffentlichungstag:
**24.05.2000 Patentblatt 2000/21**

(51) Int. Cl.[7]: **A61K 7/42**

(21) Anmeldenummer: **99120973.5**

(22) Anmeldetag: **04.11.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **19.11.1998 DE 19853288**

(71) Anmelder:
**Beiersdorf Aktiengesellschaft**
**20245 Hamburg (DE)**

(72) Erfinder:
- **Gers-Barlag, Heinrich, Dr.**
  **25495 Kummerfeld (DE)**
- **Müller, Anja**
  **23843 Rümpel (DE)**

(54) **Aluminiumkomplexe von Dibenzoylmethanderivaten und kosmetische oder dermatologische Zubereitungen, diese enthaltend**

(57) Lichtschutzwirksame Verbindungen des Aluminiums, welche das Strukturmotiv des Dibenzoylmethans enthalten, insbesondere die Struktur (I)

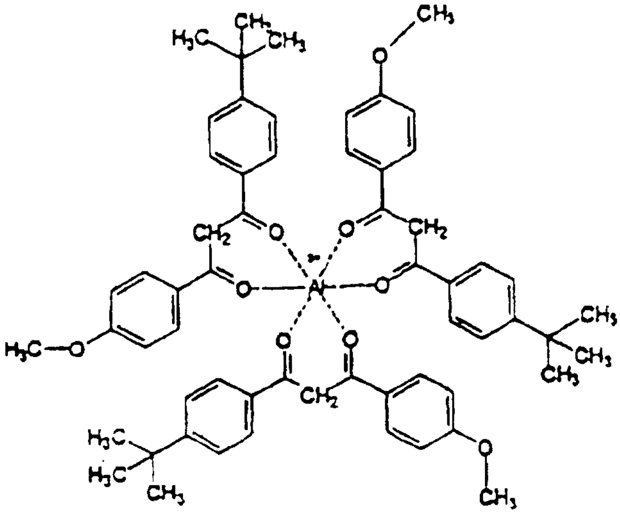

(I)

EP 1 002 522 A1

## Beschreibung

Die vorliegende Erfindung betrifft Lichtschutzformulierungen, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut „altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Vorbeugender Schutz gegen UV-A-Strahlen, beispielsweise durch Auftrag von Lichtschutzfiltersubstanzen in Form einer kosmetischen oder dermatologischen Formulierung auf die Haut, ist daher von grundsätzlicher Wichtigkeit.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Zur Prüfung der UV-A-Schutzleistung wird üblicherweise die IPD-Methode verwendet (IPD ≡ **i**mmediate **p**igment **d**arkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die Einsatzkonzentration bekannter Lichtschutzfiltersubstanzen, die insbesondere auch im UV-A-Bereich eine hohe Filterwirkung zeigen, ist häufig gerade in Kombination mit anderen als Festkörper vorliegenden Substanzen begrenzt. Es bereitet daher gewisse formulierungstechnische Schwierigkeiten, höhere Lichtschutzfaktoren bzw. UV-A-Schutzleistung zu erzielen.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind,

war es eine Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Eine bekannte und vorteilhafte Lichtschutzfiltersubstanz ist das 4-(tert.-Butyl)-4 '-methoxydibenzoylmethan, welches sich durch die Struktur

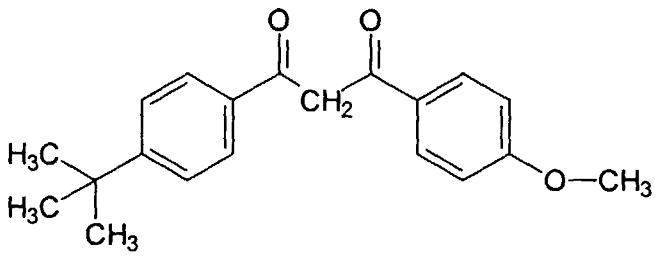

auszeichnet, und von Givaudan unter der Marke Parsol® 1789 verkauft wird

Der Hauptnachteil dieser Substanz ist eine gewisse Instabilität gegenüber UV-Strahlung, so daß es zweckmäßig ist, Zubereitungen mit einem Gehalt an dieser Substanz auch gewisse UV-Stabilisatoren einzuverleiben. Die photochemische Zersetzung von 4-(tert.-Butyl)-4'-methoxydibenzoylmethan - stellvertretend für alle im UV-Bereich absorbierenden Dibenzoylmethanderivate - folgt einer Norrish-Typ-I-Acylspaltung gemäß dem nachfolgenden Reaktionsschema:

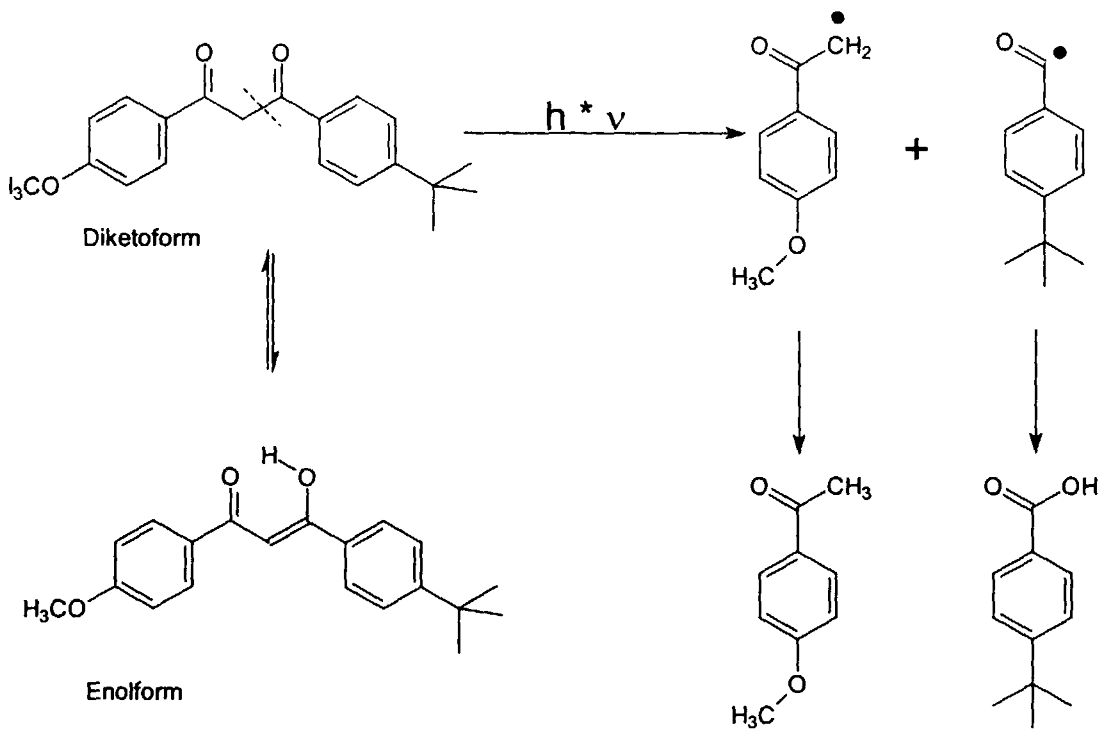


Die Reaktionsprodukte stehen als Lichtschutzfiltersubstanzen nicht mehr zur Verfügung. Es war daher ein dringender Bedarf Wege aufzuweisen, auf welchen der photolytischen Zersetzung von Dibenzoylmethanderivaten wirksam begegnet werden kann.

Beispielsweise beschreibt die deutsche Offenlegungsschrift DE-A-37 41 420 die Kombination dieses Lichtschutzfilters in bestimmtem Mengenverhältnis zu 4-Methylbenzylidencampher, welcher sich durch die Struktur

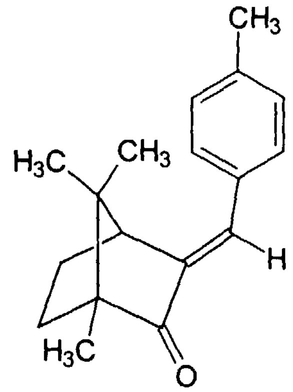

auszeichnet und von Merck unter der Marke Eusolex® 6300 verkauft wird. Die a.a.O. beschriebenen Zubereitungen zeichnen sich aber wiederum durch andere Nachteile aus, hauptsächlich formulierungstechnischer Natur.

Ein weiterer Nachteil ist, daß Dibenzoylmethanderivate recht gute Chelatbildner darstellen. Zusammen mit geringen Mengen von Aluminiumionen oder auch Zinkionen, z.B. aus der oberflächlichen Beschichtung von Mikropigmenten, welche gerade im kosmetischen Lichtschutz allgegenwärtig sind, kommt es in Emulsionen zur Kristallbildung. Die Kristalle führen zu einer Verringerung der Lichtschutzeffektivität, so daß es bisher wenig ratsam erschien, beispielsweise mit Aluminiumstearat beschichtetes Titandioxid zusammen mit Dibenzoylmethanderivaten, insbesondere 4-(tert.-Butyl)-4'-methoxydibenzoylmethan, einzusetzen.

Den Nachteilen des Standes der Technik abzuhelfen, war also Aufgabe der vorliegenden Erfindung.

Es war indes überraschend und für den Fachmann nicht vorauszusehen, daß lichtschutzwirksame Verbindungen des Aluminiums, welche das Strukturmotiv des Dibenzoylmethans enthalten, den Nachteilen des Standes der

Technik abhelfen

Überraschend und für den Fachmann nicht vorhersehbar war ferner, daß die Kombination von Dibenzoylmethanderivaten, insbesondere 4-(tert.-Butyl)-4'-methoxydibenzoylmethan, mit Aluminiumsalzen höherer Fettsäuren, insbesondere Aluminiumstearat, nicht zur Ausbildung von kristallinen Komplexen führte, sondern zu nahzu kugelförmigen Partikeln weicher Konsistenz, die sich auf der Haut gleichmäßig verteilen ließen.

Es wird davon ausgegangen, daß die die Kombination von Dibenzoylmethanderivaten, insbesondere 4-(tert.-Butyl)-4'-methoxydibenzoylmethan, mit Aluminiumsalzen höherer Fettsäuren, insbesondere Aluminiumstearat, zu Komplexverbindungen führen, welche das Strukturmotiv

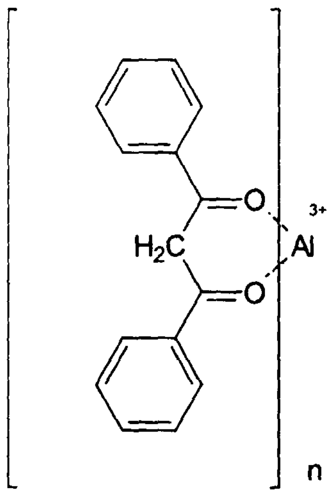

bzw.

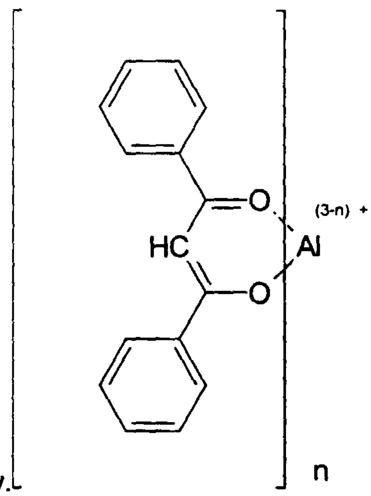

enthalten, wobei n Werte von 1 - 3 annehmen kann, insbesondere wird davon ausgegangen, daß bei der Kombination von 4-(tert.-Butyl)-4'-methoxydibenzoylmethan mit Aluminiumsalzen höherer Fettsäuren, insbesondere Aluminiumstearat, Komplexverbindungen erhalten werden, deren chemische Struktur durch die Formel

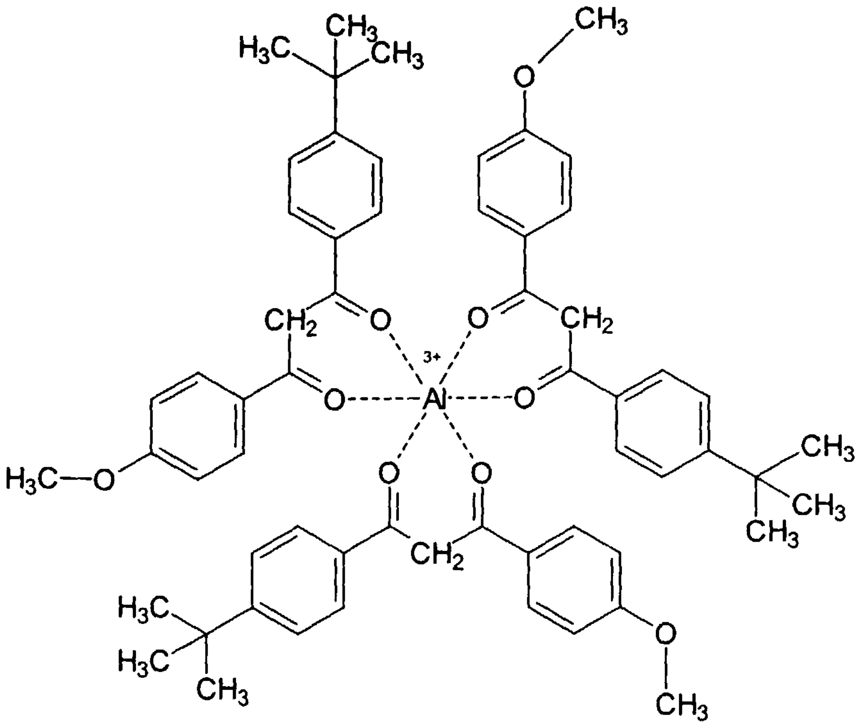

bzw.

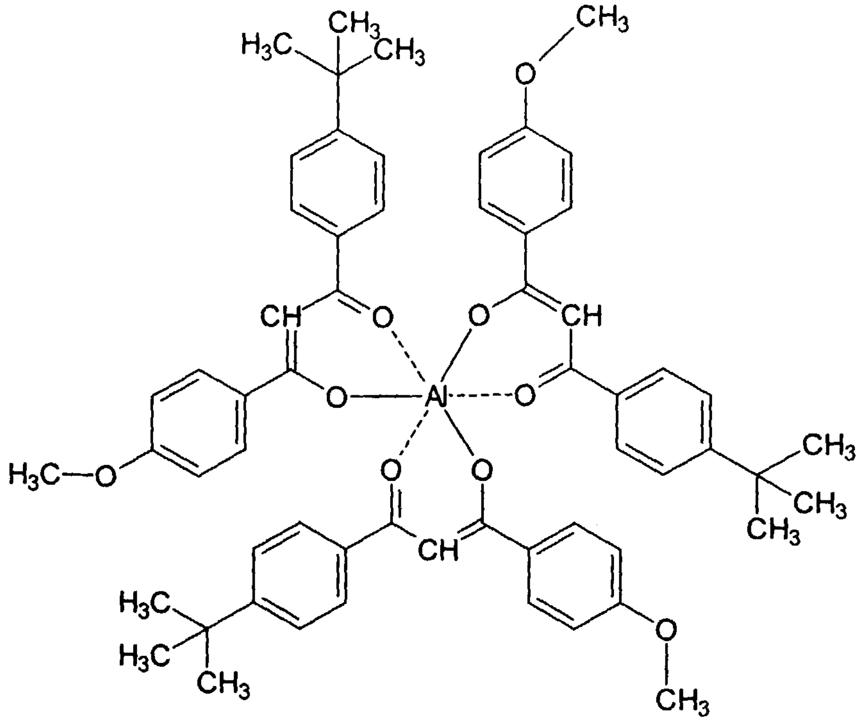

(im folgenden auch Al [Dibenzo]$_3$ bezeichnet) wiedergegeben werden.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

So bilden Komplexbildner, insbesondere Chelatoren, bekanntermaßen mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Es ist zwar an sich bekannt, daß Aluminium mit 1,3-Diketonen, wie beispielsweise dem Acetylaceton, Komplexe auszubilden imstande ist (z.B. Al-(III)-trisacetylacetonat). Gleichwohl konnte der Stand der Technik nicht den Weg zur vorliegenden Erfindung bahnen.

Die Herstellung der erfindungsgemäßen Verbindungen des Aluminiums, welche das Strukturmotiv des Dibenzoylmethans enthalten, erfolgt vorteilhaft dergestalt, daß eine Lösung oder Suspension eines Dibenzoylmethanderivates, insbesondere des 4-(tert.-Butyl)-4'-methoxydibenzoylmethans, in einem geeigneten Lösungsmittel, vorteilhaft eines Öles, insbesondere einer kosmetischen Ölphase mit einem Aluminiumsalz, beispielsweise einem gelösten oder suspendierten Aluminiumsalz, insbesondere einem in einem Öl löslichen oder dispergierbaren Aluminiumsalz, vorteilhaft einem Aluminiumsalz einer Fettsäure, insbesondere vorteilhaft Aluminiumstearat versetzt wird.

Besonders vorteilhaft im Sinne der Erfindung werden Verbindungen erhalten, die das Strukturmotiv des Dibenzoylmethans enthalten, bei einer Temperatur, die mindestens der Schmelztemperatur des Dibenzoylmethandervates entspricht, und vorteilhaft mindestens 20 °C, insbesondere 40 °C, darüberliegt, mit einem Aluminiumsalz, beispielsweise einem gelösten oder suspendierten Aluminiumsalz, insbesondere einem in einem Öl löslichen oder dispergierbaren Aluminiumsalz, vorteilhaft einem Aluminiumsalz einer Fettsäure, insbesondere vorteilhaft Aluminiumstearat, vereinigt und verrührt, bis das Dibenzoylmethandervat aufgeschmolzen bzw. gelöst vorliegt. Diese Mischung wird abgekühlt und mit den übrigen Komponenten der Fettphase vereinigt, welche gegebenenfalls daraufhin mit einer Wasserphase verrührt und gewünschtenfalls homogenisiert werden können. Nach Abkühlen auf Raumtemperatur wird eine Zubereitung mit einem Gehalt an erfindungsgemäßen Verbindungen des Aluminiums, welche das Strukturmotiv des Dibenzoylmethans enthalten, erhalten.

Bei der Vereinigung des Dibenzoylmethanderivates, insbesondere des 4-(tert.-Butyl)-4'-methoxydibenzoylmethans, mit dem Aluminiumsalz wird vorteilhaft eine Temperatur im Bereich von ca. 85 °C - ca. 150 °C, insbesondere

im Bereich von ca. 100 °C - ca. 140 °C, bevorzugt im Bereich von ca. 110 °C - ca. 130 °C gewählt und hernach auf Raumtemperatur abgekühlt.

Es kann dabei von Vorteil sein, gleichzeitig mit dem oder den Dibenzoylmethanderivaten bzw. dem Aluminiumsalz oder den Aluminiumsalzen andere übliche lipophile bzw. in Öl lösliche bzw. in Öl dispergierbare Bestandteile einer kosmetischen Ölphase vorzulegen, da sich die erfindungsgemäß erhältlichen kugelförmigen Partikeln weicher Konsistenz sich gleichsam wie Koazervate verhalten, denen auf diese einfache Weise weitere vorteilhafte kosmetische und/oder dermatologische Wirkstoffe einverleibt werden können.

Es ist möglich und vorteilhaft, die Größe der entstehenden Partikel der lichtschutzwirksame Verbindung bzw. der Verbindungen des Aluminiums, welche das Strukturmotiv des Dibenzoylmethans enthalten, dadurch zu steuern, daß eine Steigerung der Konzentration des Aluminiumsalzes zu größeren Partikeln führt. Es können Partikel eines Durchmessers von typischerweise bis zu etwa 1 mm erhalten werden, wobei aber auch Partikelgrößen im µm-Bereich und Partikelgrößen größer als 1 mm erhältlich sind.

Bei Befolgen der erfindungsgemäßen Lehre sind Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Insbesondere die Stabilität von 4-(tert.-Butyl)-4'-methoxydibenzoylmethan gegen die Zersetzung unter UV-Licht wird drastisch erhöht.

Die Gesamtmenge an Dibenzoylmethanen, insbesondere 4-(tert.-Butyl)-4'-methoxydibenzoylmethan in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Erfindungsgemäße kosmetische und dermatologische Zubereitungen enthalten außerdem vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Die anorganischen Pigmente liegen erfindungsgemäß in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase der erfindungsgemäßen Zubereitungen mit einem Gehalt an einer oder mehreren erfindungsggemäßen lichtschutzwirksame Verbindungen des Aluminiums, welche das Strukturmotiv des Dibenzoylmethans enthalten kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12\text{-}15}$-Alkylbenzoat, Capryl-Caprinsäure-triglycerid, Dicaprylylether.

Besonders vorteilhaft sind Mischungen aus $C_{12\text{-}15}$-Alkylbenzoat und 2-Ethylhexylisostearat, Mischungen aus $C_{12\text{-}15}$-Alkylbenzoat und Isotridecylisononanoat sowie Mischungen aus $C_{12\text{-}15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Es ist von Vorteil, die Herstellung der erfindungsgemäßen lichtschutzwirksamen Verbindungen des Aluminiums, welche das Strukturmotiv des Dibenzoylmethans enthalten, in einer Lipidphase vorzunehmen, welche bereits als Grundlage für die spätere Lipidphase der kosmetischen bzw. dermatologischen Zubereitungen dienen kann.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl-oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropyl-methylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Es ist erfindungsgemäß bevorzugt, den erfindungsgemäßen kosmetischen oder dermatologischen Zubereitungen, solche Wirkstoffe enthaltend, weitere Komplexbildner zuzufügen.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Emulsionen entsprechend der vorliegenden Erfindungen enthalten einen oder mehrere Emulgatoren, insbesondere vorteilhaft gewählt aus der Gruppe Glycerylstearat im Gemisch mit Ceteareth-20; Sorbitanstearat; Sorbitanoleat; Ceteareth-25; Ceteareth-6 im Gemisch mit Stearylalcohol; Cetylstearylalcohol im Gemisch mit PEG-40-Ricinusöl und Natriumcetylstearylsulfat; Triceteareth-4 Phosphat; Glycerylstearat; Natriumcetylstearylsulfat; Lecithin; Trilaureth-4 Phosphat; Laureth-4 Phosphat; Stearinsäure; Propylenglycolstearat SE; PEG-25-hydriertes Ricinusöl; PEG-54-hydriertes Ricinusöl; PEG-6 Caprylsäure/Caprinsäureglyceride; Sorbitanstearat; Glyceryloleat im Gemisch mit Propylenglycol; PEG-9-Stearat; Glyceryllanolat; Ceteth-2; Ceteth-20; Polysorbat 60; Lanolin; Glycerylstearat im Gemisch mit PEG-100 Stearat; Glycerylmyristat; mikrokristallines Wachs (Cera microcristallina) im Gemisch mit Paraffinöl (Paraffinum liquidum), Ozokerit, hydriertem Ricinusöl, Glyceryl Isostearat und Polyglyceryl-3-Oleat; Glyceryllaurat; PEG-40-Sorbitanperoleat; Laureth-4; Ceteareth-3; Wollwachssäuregemische; Isostearylglycerylether; Cetylstearylalkohol im Gemisch mit Natrium Cetylstearylsulfat; Wollwachsalkoholgemische; Laureth-23; Steareth-2; Glycerylstearat im Gemisch mit PEG-30 Stearat; PEG-40-Stearat; Glycol Distearat; PEG-22-Dodecyl Glycol Copolymer; Polyglyceryl-2-PEG-4 Stearat; Pentaerythrithyl-isostearat; Polyglyceryl-3 Diisostearat; Ceteareth-20; Sorbitan Oleat im Gemisch mit hydriertem Ricinusöl, Bienenwachs (Cera alba) und Stearinsäure; Natriumdihydroxycetylphosphat im Gemisch mit Isopropylhydroxycetylether; Methylglucosesesquistearat; Steareth-10; PEG-20-Stearat; Steareth-2 im Gemisch mit PEG-8 Distearat; Steareth-21; Steareth-20; Isosteareth-20; Methylglucosedioleat; PEG-7-hydriertes Ricinusöl; Sorbitanoleat im Gemisch mit PEG-2-hydriertem Ricinusöl, Ozokerit und hydriertem Ricinusöl; Sorbitanisostearat im Gemisch mit PEG-2-hydriertem Ricinusöl, Ozokerit und hydriertem Ricinusöl; PEG-45/ Dodecylglycol-Copolymer; Methoxy-PEG-22/Dodecylglycol-Copolymer; hydrierte Cocosfettsäureglyceride; Polyglyceryl-4-Isostearat; PEG-40-Sorbitanperoleat; PEG-40-Sorbitanperisostearat; PEG-20-Glycerylstearat; PEG-20-Glycerylstearat; PEG-8-Bienenwachs; Laurylmethiconcopolyol; Cetyldimethiconcopolyol; Polyglyceryl-2-laurat; Isostearyldiglycerylsuccinat; Stearamidopropyl-PG-dimoniumchloridphosphat; PEG-7-hydriertes Ricinusöl; Glycerylstearat, Ceteth-20; Triethylcitrat; PEG-20-Methylglucosesesquistearat; Ceteareth-12; Paraffinöl (Paraffinum liquidum); Glycerylstearatcitrat; Cetylphosphat; Sorbitansesquioleat; Acrylat/$C_{10-30}$-Alkylacrylat-Crosspolymer; Sorbitanisostearat; Methylglucosesesquistearat; Triceteareth-4-Phosphat; Trilaureth-4-Phosphat; Polyglycerylmethylglucosedistearat; Poloxamer 101; Kaliumcetylphosphat; Isosteareth-10; Polyglyceryl-2-sesquiisostearat; Ceteth-10; Polyglyceryl-2 Dipolyhydroxystearat; Oleth-20; Isoceteth-20; Glycerylisostearat; Polyglyceryl-3-Diisostearat; Glycerylstearat im Gemisch mit Ceteareth-20, Ceteareth-12, Cetylstearylalcohol und Cetylpalmitat; Cetylstearylalcohol im Gemisch mit PEG-20 Stearat; Glycerylstearat; PEG-30-Stearat.

Erfindungsgemäße Zubereitungen können sich ferner durch einen Gehalt an Tensiden auszeichnen. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise $-COO^-$, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

$RNH_2^+CH_2CH_2COOH\ X^-$ (bei pH=2) $X^-$ = beliebiges Anion, z.B. $Cl^-$
$RNH_2^+CH_2CH_2COO^-$ (bei pH=7)
$RNHCH_2CH_2COO^-\ B^+$ (bei pH=12) $B^+$ = beliebiges Kation, z.B. $Na^+$

Typisch für nicht-ionische Tenside sind Polyether-Ketten. Nicht-ionische Tenside bilden in wäßrigem Medium keine Ionen.

A. Anionische Tenside

Vorteilhaft zu verwendende anionische Tenside sind

Acylaminosäuren (und deren Salze), wie

1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,

4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,
Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium $C_{12-14}$ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat

sowie
Schwefelsäureester, wie

1. Alkylethersulfat, beispielsweise Natrium-, Ammonium-, Magnesium-, MIPA-, TIPA- Laurethsulfat, Natriummyrethsulfat und Natrium $C_{12-13}$-Parethsulfat,
2. Alkylsulfate, beispielsweise Natrium-, Ammonium- und TEA-Laurylsulfat.

B. Kationische Tenside

Vorteilhaft zu verwendende kationische Tenside sind

1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Aryl-gruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

C. Amphotere Tenside

Vorteilhaft zu verwendende amphotere Tenside sind

1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

2. N-Alkylaminosäuren, beispielsweise Aminopropylalkylglutamid, Alkylaminopropionsäure, Natriumalkylimidodipropionat und Lauroamphocarboxyglycinat.

D. Nicht-ionische Tenside

Vorteilhaft zu verwendende nicht-ionische Tenside sind

1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Die Gesamtmenge an Tensiden in den fertigen kosmetischen oder dermatologischen Zubereitungen gemäß der Erfindung wird vorteilhaft aus dem Bereich von 0,001 - 50 Gew.-%, bevorzugt 0,1- 10,0 Gew.-%, insbesondere 0,5 - 5,0 Gew.-% gewählt, bezogen auf das Gesamtgewicht der Zubereitungen.

Erfindungsgemäße kosmetische und dermatologische Zubereitungen enthalten vorteilhaft, wenngleich nicht zwingend, weitere anorganische Pigmente, welche röntgenamorph oder nichtröntgenamorph sein können.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen gemäß der Erfindung werden röntgenamorphe Oxidpigmente vorteilhaft als Verdickungs-und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt, sie können aber auch der Verstärkung der Lichtschutzleistung dienen.

Bekannte und vorteilhaft in Zubereitungen gemäß der Erfindung zu verwendende röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß vorteilhaft, wenngleich nicht zwingend, enthalten kosmetische oder dermatologische Lichtschutzzubereitungen 0,1 bis 20 Gew.-%, bevorzugt 0,5 bis 10 Gew.-%, ganz besonders bevorzugt 1 bis 5 Gew.-% röntgenamorphe Oxidpigmente.

Ferner vorteilhaft können Zubereitungen gemäß der Erfindung anorganische Pigmente, insbesondere Mikropigmente enthalten, welche nichtröntgenamorph sind, auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Derlei Pigmente schirmen UV-Strahlung, auch UVA-Strahlung ab und sind im Sinne der Erfindung als UV-Filtersubstanzen anzusehen.

Die nichtröntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer

Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Bekannte und im Sinne der vorliegenden Erfindung vorteilhaft zu verwendende Lichtschutzfiltersubstanzen sind Dibenzoylmethanderivate, beispielsweise 5-Isopropyldi-benzoylmethan (CAS-Nr. 63250-25-9), welches sich durch die Struktur

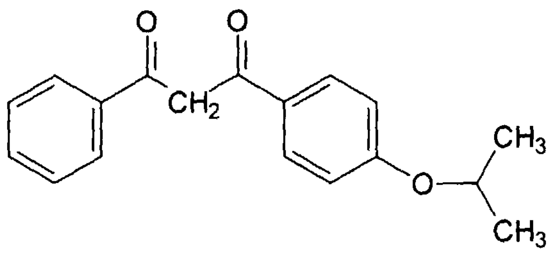

auszeichnet und von Merck unter der Marke Eusolex® 8020 verkauft wird, sowie das 4-(tert.-Butyl)-4'-methoxydibenzoylmethan (CAS-Nr. 70356-09-1), welches sich durch die Struktur

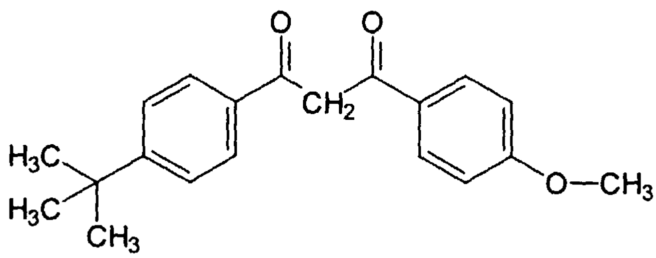

auszeichnet, und von Givaudan unter der Marke Parsol® 1789 verkauft wird.

Eine weitere bekannte und im Sinne der vorliegenden Erfindung vorteilhaft zu verwendende Lichtschutzfiltersubstanz ist der 4-Methylbenzylidencampher, welcher sich durch die Struktur

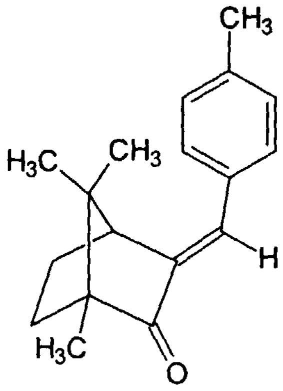

auszeichnet und von Merck unter der Marke Eusolex® 6300 verkauft wird. 4-Methylbenzylidencampher ist eine äußerst vorteilhafte, unter Normalbedingungen als Festkörper vorliegende Lichtschutzfiltersubstanz und zeichnet sich an sich durch gute UV-Filtereigenschaften aus.

Auch andere Benzylidencampherderivate sind im Sinne der vorliegenden Eerfindung vorteilhaft einzusetzende Lichtschutzfiltersubstanzen, z.B. der Benzylidencampher, welcher sich durch die Struktur

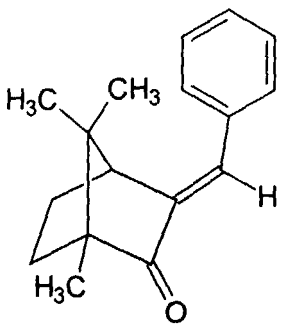

auszeichnet und von der Gesellschaft Induchem unter der Marke Unisol® S22 verkauft wird.

Ein weiterer vorteilhafter UV-Filter ist der 4,4',4''-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin.

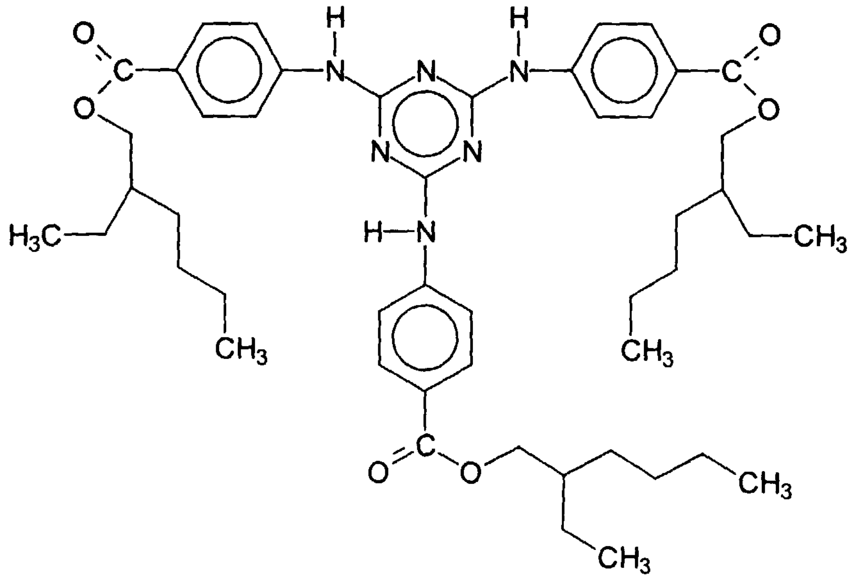

Diese UVB-Filtersubstanz wird von der BASF Aktiengesellschaft unter der Warenbezeichnung UVINUL® T 150 vertrieben und zeichnet sich durch gute UV-Absorptionseigenschaften aus. Zwischenzeitlich wurden von verschiedenen Autoren auch andere UV-Filtersubstanzen beschrieben, welche das Strukturmotiv

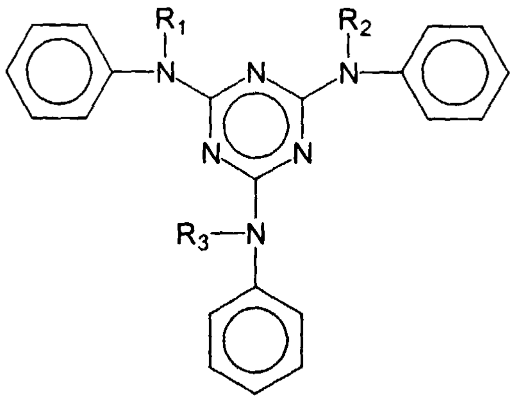

aufweisen.

Auch andere als Festkörper vorliegende UV-Filtersubstanzen sind im Sinne der vorliegenden Erfindung vorteilhaft einzusetzen. So werden in der EP-A- 570 838 s-Triazinderivate beschrieben, deren chemische Struktur durch die generische Formel

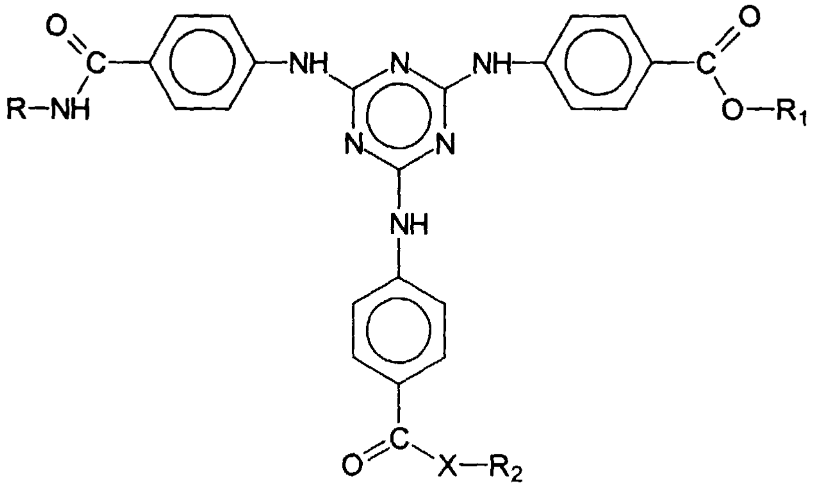

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

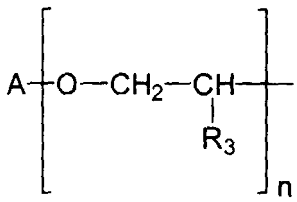

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

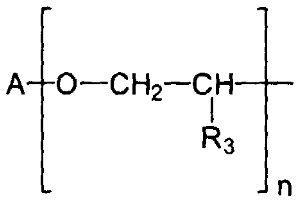

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

wenn X ein Sauerstoffatom darstellt.

Ein Beispiel für solche symmetrisch substituierte s-Triazine stellt das Dioctylbutylamidotriazon dar, dessen chemische Struktur durch die Formel

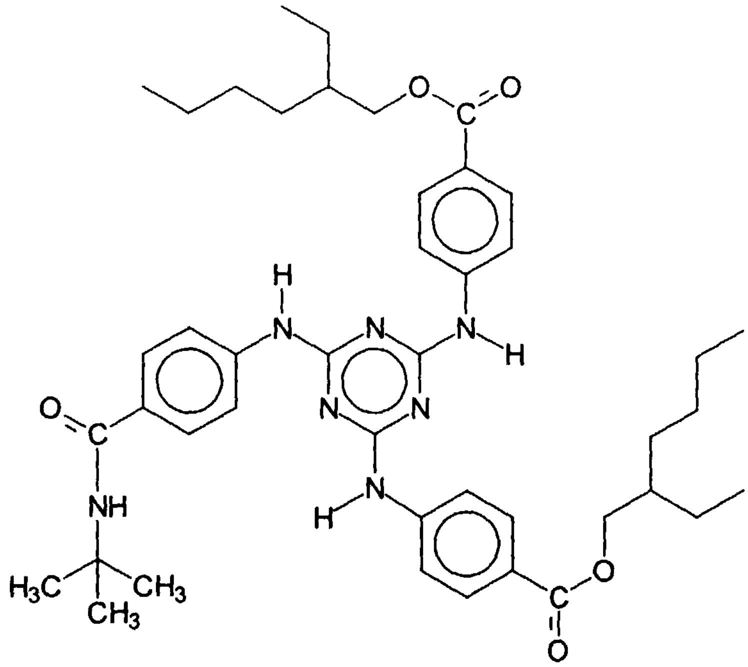

wiedergegeben wird.

Auch andere UV-Filtersubstanzen, deren Einarbeitung in kosmetische oder dermatologische Lichtschutzformulierungen zumindest gewisse Probleme aufweist, sind bekannt. So werden in der EP-A-775 698 Bis-Resorcinyltriazinderivate beschrieben, deren chemische Struktur durch die generische Formel

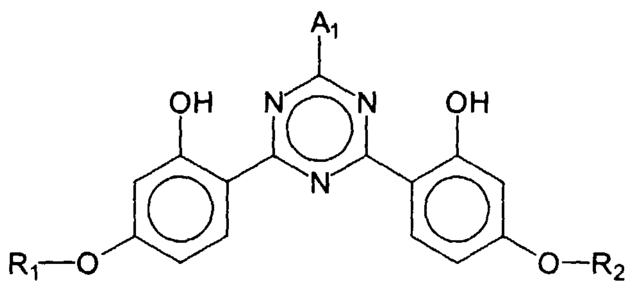

wiedergegeben wird, wobei $R_1$ , $R_2$ und $A_1$ verschiedenste organische Reste repräsentieren.

Vorteilhafte Bis-Resorcinyltriazinderivate sind ferner beispielsweise folgende Verbindungen:

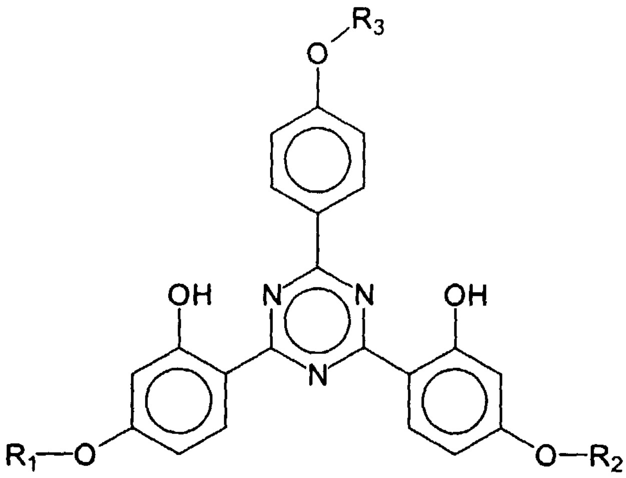

wobei $R_3$ ein Wasserstoffatom oder eine verzweigte oder unverzweigte Alkylgruppe mit 1 bis 10 Kohlenstoffatomen darstellt und $R_1$ und $R_2$ die vorgenannten Bedeutungen haben, insbesondere das 2,4-Bis-{[4-(2-Ethyl-hexyloxy)-2-hydroxy]-phenyl}-6-(4-methoxyphenyl)-1,3,5-triazin, welches durch folgende Struktur gekennzeichnet ist:

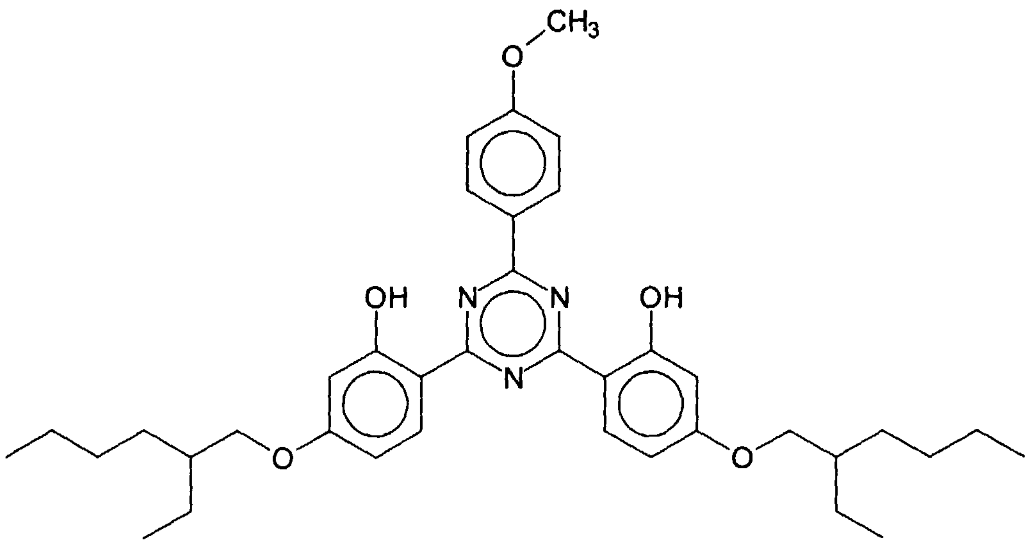

Ferner vorteilhaft ist das 2,4-Bis-{[4-(3-sulfonato)-2-hydroxy-propyloxy)-2-hydroxy]-phenyl}-6-(4-methoxyphenyl)-1,3,5-triazin Natriumsalz, welches durch folgende Struktur gekennzeichnet ist:

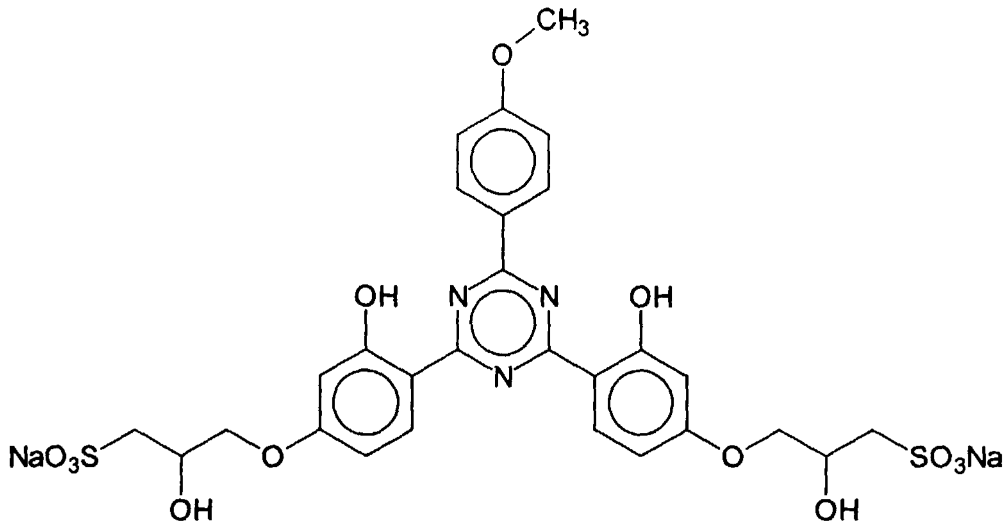

Ferner vorteilhaft ist das 2,4-Bis-{[4-(3-(2-Propyloxy)-2-hydroxy-propyloxy)-2-hydroxy]-phenyl}-6-(4-methoxyphenyl)-1,3,5-triazin, welches durch folgende Struktur gekennzeichnet ist:

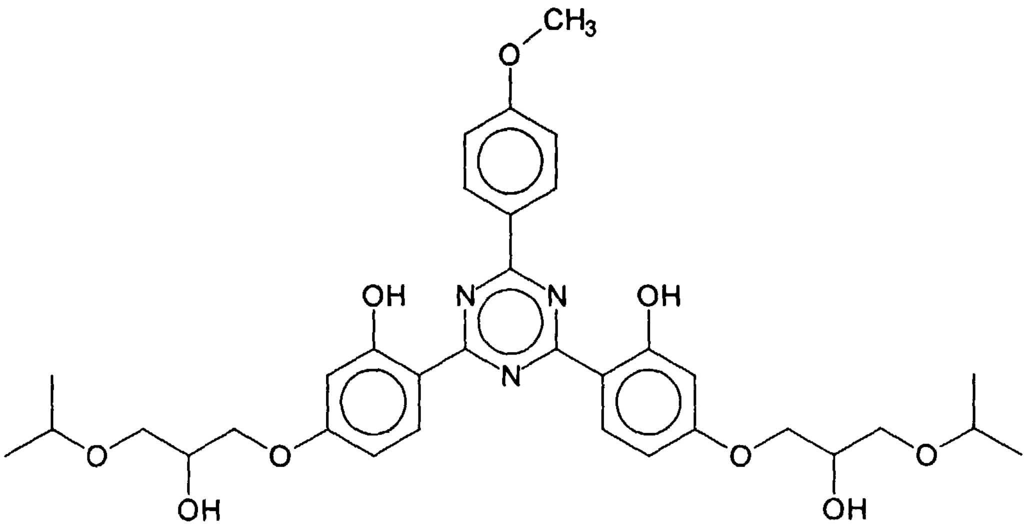

Ferner vorteilhaft ist das 2,4-Bis-{[4-(2-ethyl-hexyloxy)-2-hydroxy]-phenyl}-6-[4-(2-methoxyethyl-carboxyl)-phenylamino]-1,3,5-triazin, welches durch folgende Struktur gekennzeichnet ist:

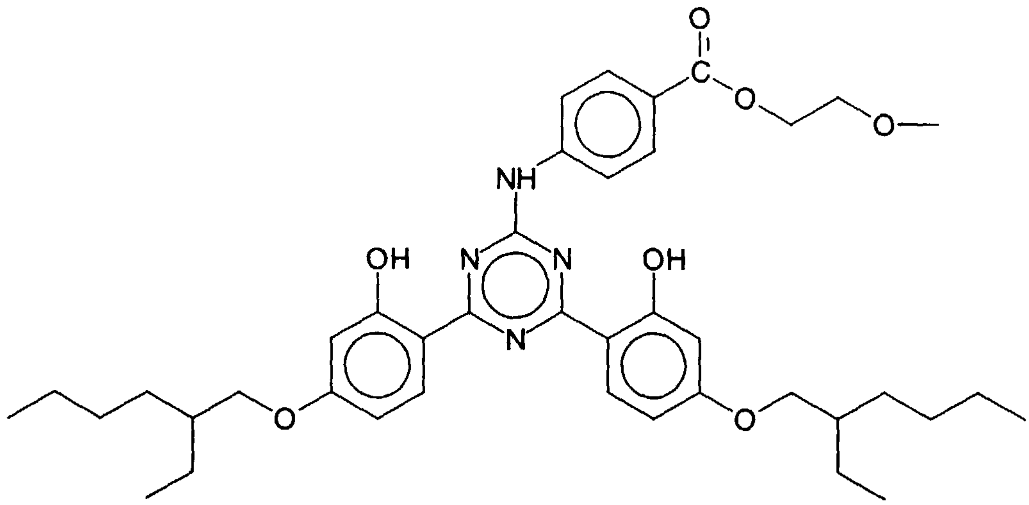

Ferner vorteilhaft ist das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, welches durch folgende Struktur gekennzeichnet ist:

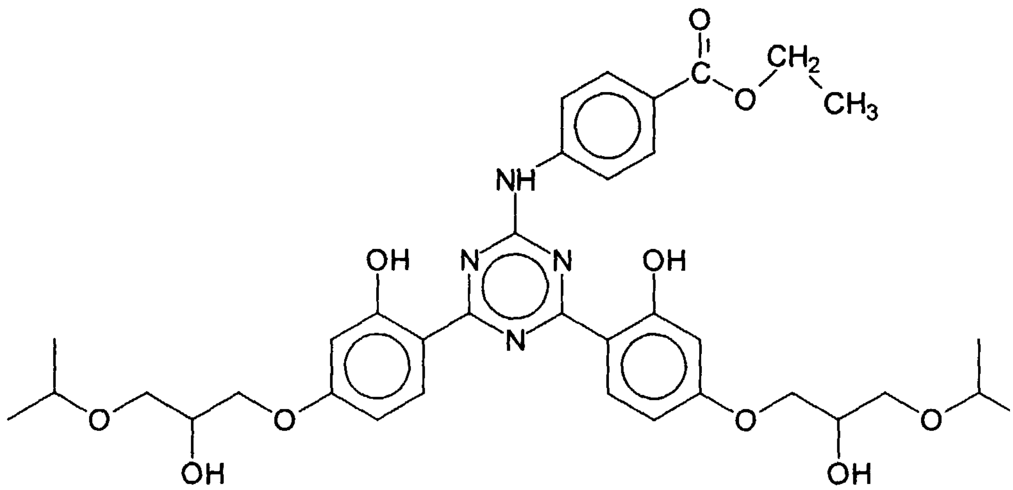

Ferner vorteilhaft ist das 2,4-Bis-{[4-(2-ethyl-hexyloxy)-2-hydroxy]-phenyl}-6-(1-methyl-pyrrol-2-yl)-1,3,5-triazin, welches durch folgende Struktur gekennzeichnet ist:

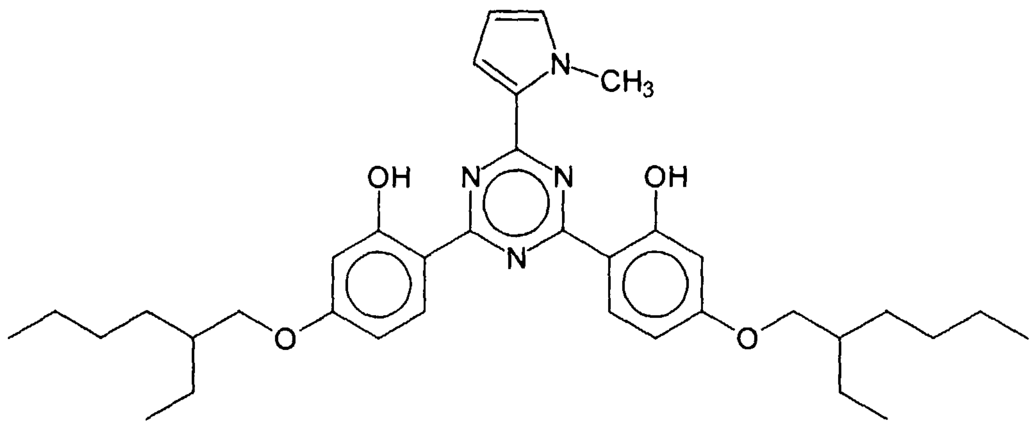

Ferner vorteilhaft ist das 2,4-Bis-{[4-tris(trimethylsiloxy-silylpropyloxy)-2-hydroxy]-phenyl}-6-(4-methoxyphenyl)-1,3,5-triazin, welches durch folgende Struktur gekennzeichnet ist:

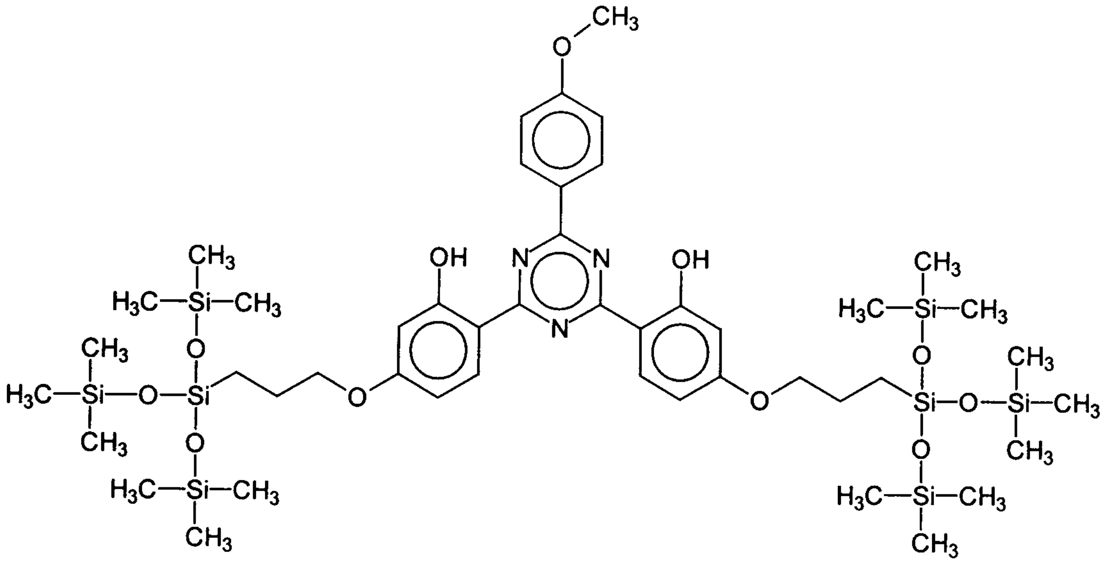

Ferner vorteilhaft ist das 2,4-Bis-{[4-(2"-methylpropenyloxy)-2-hydroxy]-phenyl}-6-(4-methoxyphenyl)-1,3,5-triazin, welches durch folgende Struktur gekennzeichnet ist:

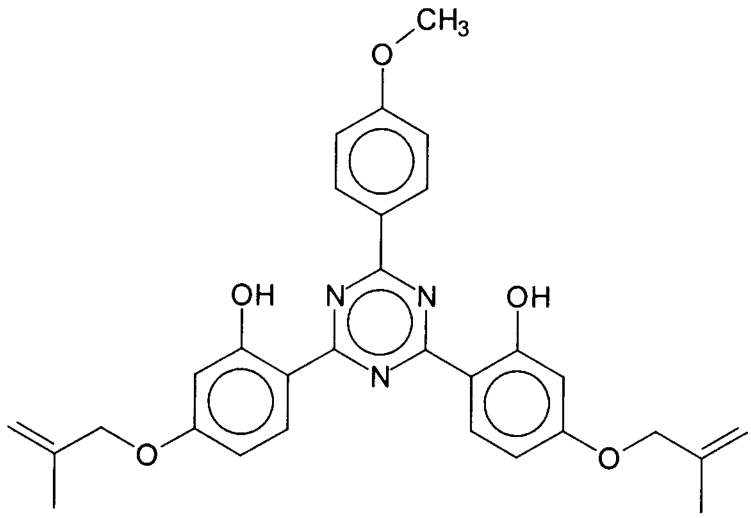

Ferner vorteilhaft ist das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin, welches durch folgende Struktur gekennzeichnet ist:

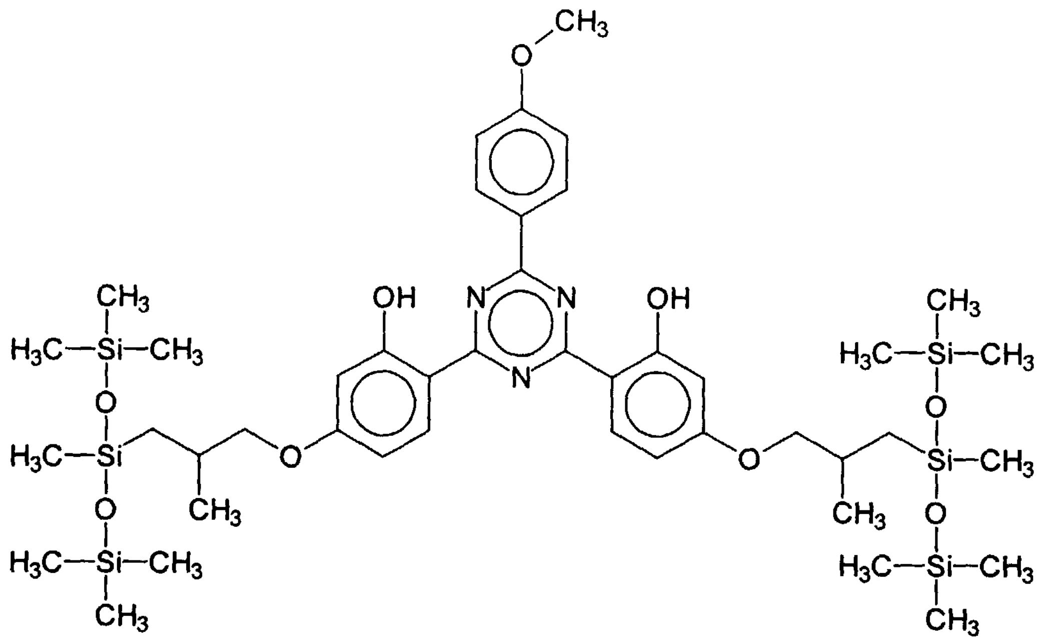

Noch ein weiterer vorteilhafter UV-Filter ist das 2,2'-Methylen-bis-(6-(2H-benzotriazol-2-yl)-4-(1,1,3,3-tetramethylbutyl)-phenol), welches durch die chemische Strukturformel

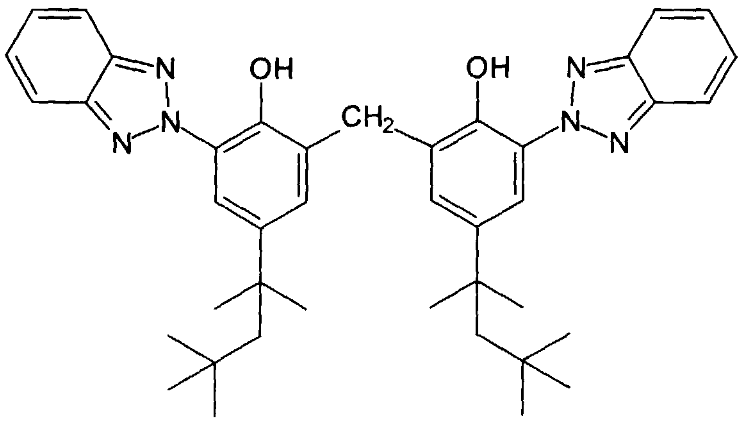

gekennzeichnet ist.

Insbesondere vorteilhaft werden der oder die UV-Filter in den Lichtschutzzubereitungen gemäß der vorliegenden Erfindung aus der Gruppe der Triazinderivate gewählt, insbesondere gewählt aus der Gruppe

- 4,4',4"-(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl}-6-(4-methoxyphenyl)-1,3,5-triazin Natriumsalz,
- 2,4-Bis-{[4-(3-(2-Propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethylcarboxyl)-phenylamino]-1,3,5-triazin,
- 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2"-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ferner ist vorteilhaft, wenn wenigstens einer der UV-Filter in den Lichtschutzzubereitungen gemäß der vorliegenden Erfindung das 2,2'-Methylen-bis-(6-(2H-benzotriazol-2-yl)-4-(1,1,3,3-tetramethylbutyl)-phenol) ist.

Vorteilhafte UV-A-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter sind beispielsweise Bis-Resorcinyltriazinderivate mit der folgenden Struktur:

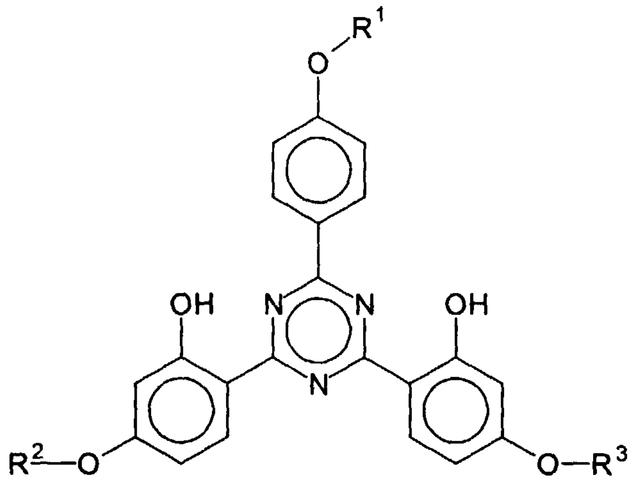

wobei $R^1$, $R^2$ und $R^3$ unabhängig voneinander gewählt werden aus der Gruppe der verzweigten und unverzweigten

Alkylgruppen mit 1 bis 10 Kohlenstoffatomen bzw. ein einzelnes Wasserstoffatom darstellen. Insbesondere bevorzugt ist das 2,4-Bis-{[4-(2-Ethyl-hexyloxy)-2-hydroxy]-phenyl}-6-(4-methoxyphenyl)-1,3,5-triazin, welches durch folgende Struktur gekennzeichnet ist:

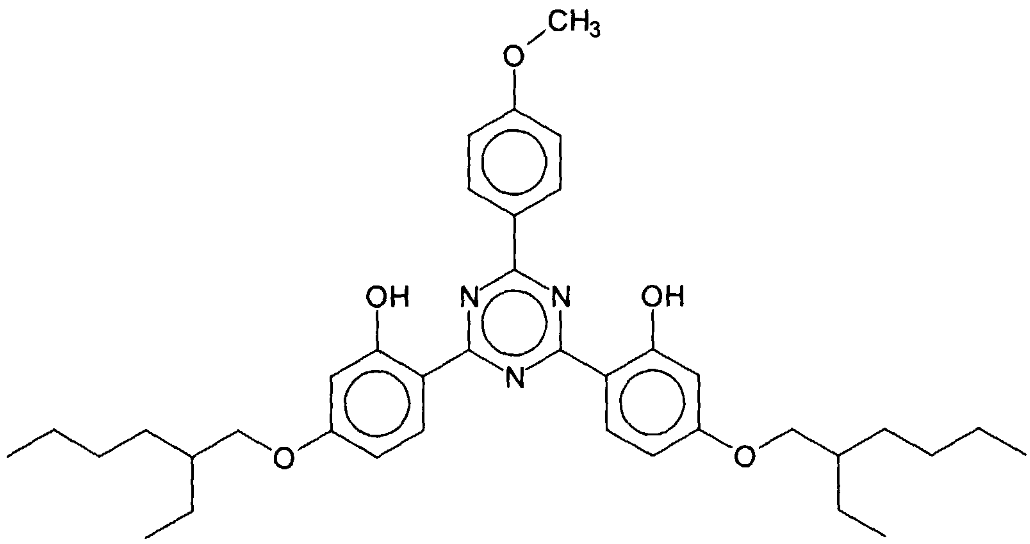

Kosmetische und dermatologische Formulierungen im Sinne der Erfindung enthalten vorteilhaft einen oder mehrere übliche UV-A-Filter als Einzelsubstanzen oder in beliebigen Gemischen untereinander, in der Lipidphase und/oder in der wäßrigen Phase.

Die Gesamtmenge an UV-A-Filtersubstanzen in den fertigen kosmetischen oder dermatologischen Zubereitungen wird vorteilhaft aus dem Bereich von 0,1 bis 30 Gew.-%, bevorzugt 0,1 bis 10,0 Gew.-%, insbesondere 0,5 bis 5,0 Gew.-% gewählt, bezogen auf das Gesamtgewicht der Zubereitungen.

Solche UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Vorteilhafte wasserlösliche UVB-Filtersubstanzen sind z.B.:

- Salze der 2-Phenylbenzimidazol-5-sulfonsäure wie ihr Natrium-, Kalium-oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Vorteilhaft sind auch Zubereitungen gemäß der Erfindung, dadurch gekennzeichnet, daß die kosmetischen oder dermatologischen Lichtschutzzubereitungen als UV-A-Filtersubstanz ein oder mehrere wasserlösliche UV-A-Filtersubstanzen, besonders wasserlösliche UV-A-Filtersubstanzen gewählt aus der Gruppe Phenylen-1,4-bis-(2-benzimidazyl)-3,3'-5,5'-tetrasulfonsäure und/oder 1,4-di(2-oxo-10-Sulfo-3-bornylidenmethyl)-Benzol und/oder ihre Salze, besonders die entsprechenden Natrium-, Kalium- oder Triethanolammonium-Salze und/oder die entprechenden 10-Sulfato-verbindungen, insbesondere das Phenylen- 1,4-bis-(2-benzimidazyl)-3,3'-5,5'-tetrasulfonsäure- bis -natriumsalz, enthalten.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Vorteilhaft sind daher auch Zubereitungen gemäß der Erfindung, enthaltend polymergebundene oder polymere UV-Filtersubstanzen, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Die Gesamtmenge an wasserlöslichen UV-Filtersubstanzen in den fertigen kosmetischen oder dermatologischen Zubereitungen gemäß der Erfindung wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an öllöslichen UV-Filtersubstanzen in den fertigen kosmetischen oder dermatologischen Zubereitungen gemäß der Erfindung wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzophenonen, insbesondere Benzophenon-1, Benzophenon-2, Benzophenon-3, Benzophenon-4, Benzophenon-6, Benzophenon-8, Benzophenon-9 und/oder Benzophenon-11, in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Phenylbenzimidazol-5-sulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Methyl-5-(2-oxo-3-bornylidenmethyl)benzolsulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bornylidenmethyl-10-Sulfonsäure bzw. deren Salzen in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4,4',4''-(1,3,5-Triazin-2,4,6-triyltriimino)-tris-benzoesäure-tris(2-ethylhexylester) in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-Methylbenzylidencampher in den fertigen kosmetischen oder dermatologischen Zubereitungen gemäß der Erfindung wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Ethylhexyl-p-methoxy-cinnamat in den fertigen kosmetischen oder dermatologischen Zubereitungen gemäß der Erfindung wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

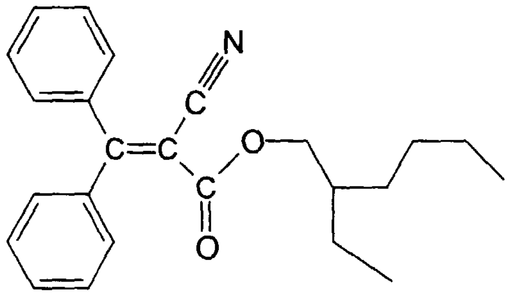

Die Gesamtmenge an Ethylhexyl-2-cyano-3,3-diphenylacrylat in den fertigen kosmetischen oder dermatologischen Zubereitungen gemäß der Erfindung wird, falls die Gegenwart dieser Substanz erwünscht ist, vorteilhaft aus dem Bereich von 0,1 - 15,0 Gew.-%, bevorzugt 0,5 - 10,0 Gew.-% gewählt, bezogen auf das Gesamtgewicht der Zubereitungen.
Ferner kann es gegebenenfalls von Vorteil sein, weitere UVA- und/oder UVB-Filter in kosmetische oder dermatologische Zubereitungen gemäß der Erfindung einzuarbeiten, beispielsweise bestimmten Salicylsäurederivaten wie 4-Isopropylbenzylsalicylat, 2-Ethylhexylsalicylat (=Octylsalicylat), Homomenthylsalicylat.
Die Gesamtmenge an einem oder mehreren Salicylsäurederivaten in den fertigen kosmetischen oder dermatologischen Zubereitungen gemäß der Erfindung wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.
Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1**

O/W-Emulsion

| | Gew.-% |
|---|---|
| Stearinsäure | 1,50 |
| Glycerinmonostearat | 3,00 |
| Caprylsäure/Caprinsäuretriglyceride | 10,00 |
| Dicaprylylether | 5,00 |
| Dimethicone | 2,00 |
| Hydriertes Polyisobuten | 2,00 |
| Vitamin E-Acetat | 0,50 |
| Octyltriazon | 2,00 |
| Methylbenzylidencampher | 4,00 |
| Butylmethoxydibenzoylmethan | 2,00 |
| Titandioxid | 1,00 |
| Aluminiumstearat | 2,00 |

(fortgesetzt)

| | Gew.-% |
|---|---|
| Konservierung | 0,50 |
| Glycerin | 3,00 |
| Xanthangummi | 0,30 |
| Natronlauge 45% | 0,50 |
| Wasser | ad 100,00 |

Die Caprylsäure/Caprinsäuretriglyceride, das Aluminiumstearat und das Butylmethoxydibenzoylmethan werden vereinigt und bei 120 °C verrührt, bis das Butylmethoxydibenzoylmethan bzw. das Aluminiumstearat aufgeschmolzen bzw. gelöst vorliegen. Diese Mischung wird auf 80 °C abgekühlt und mit den übrigen Komponenten der Fettphase vereinigt, welche daraufhin mit der Wasserphase verrührt werden. Nach Homogenisieren und Abkühlen auf Raumtemperatur wird eine O/W-Emulsion mit einem Gehalt an Al $[Dibenzo]_3$-Kügelchen erhalten.

__**Beispiel 2**__

O/W-Emulsion

| | Gew.-% |
|---|---|
| Sorbitanstearat | 3,00 |
| Polyglyceryl-3 Methylglucose Distearat | 1,50 |
| Octyldodecanol | 10,00 |
| Dicaprylylether | 5,00 |
| Mineralöl | 5,00 |
| Ricinusöl | 2,00 |
| Butylenglycoldicaprylat/Caprat | 5,00 |
| Vitamin E-Acetat | 0,50 |
| Octyltriazon | 4,00 |
| Methylbenzylidencampher | 4,00 |
| Butylmethoxydibenzoylmethan | 3,00 |
| Aluminiumstearat | 4,00 |
| Konservierung | 0,50 |
| Glycerin | 10,00 |
| Xanthangummi | 0,20 |
| Pemulen TR1 | 0,10 |
| Phenylbenzimidazolsulfonsäure | 2,00 |
| Natronlauge 45% | 1,20 |
| Wasser | ad 100,00 |

Das Mineralöl, das Octyldodecanol, das Aluminiumstearat und das Butylmethoxydibenzoylmethan werden vereinigt und bei 120 °C verrührt, bis das Butylmethoxydibenzoylmethan bzw. das Aluminiumstearat aufgeschmolzen bzw. gelöst vorliegen. Diese Mischung wird auf 80 °C abgekühlt und mit den übrigen Komponenten der Fettphase vereinigt, welche daraufhin mit der Wasserphase verrührt werden. Nach Homogenisieren und Abkühlen auf Raumtemperatur wird eine O/W-Emulsion mit einem Gehalt an Al [Dibenzo]$_3$-Kügelchen erhalten.

**Beispiel 3**

W/O-Emulsion

| | Gew.-% |
|---|---|
| Polyglyceryl-2 Dipolyhydroxystearat | 5,00 |
| Dimethicone | 2,00 |
| Mineralöl | 10,00 |
| Isohexadekan | 5,00 |
| Butylenglycoldicaprylat/Caprat | 10,00 |
| Octyltriazon | 2,00 |
| Methylbenzylidencampher | 2,00 |
| Butylmethoxydibenzoylmethan | 2,00 |
| Titandioxid | 4,00 |
| Aluminiumstearat | 1,00 |
| Konservierung | 0,50 |
| Glycerin | 5,00 |
| $MgSO_4$ | 1,00 |
| Wasser | ad 100,00 |

Das Mineralöl, das Aluminiumstearat und das Butylmethoxydibenzoylmethan werden vereinigt und bei 120 °C verrührt, bis das Butylmethoxydibenzoylmethan bzw. das Aluminiumstearat aufgeschmolzen bzw. gelöst vorliegen. Diese Mischung wird auf 80 °C abgekühlt und mit den übrigen Komponenten der Fettphase vereinigt, welche daraufhin mit der Wasserphase verrührt werden. Nach Homogenisieren und Abkühlen auf Raumtemperatur wird eine W/O-Emulsion mit einem Gehalt an Al [Dibenzo]$_3$-Kügelchen erhalten.

**Beispiel 4**

W/O- Emulsion

| | Gew. -% |
|---|---|
| PEG-30 Dipolyhydroxystearat | 4,00 |
| Caprylsäure/Caprinsäuretriglyceride | 5,00 |
| Octyldodecanol | 5,00 |
| Dicaprylylether | 5,00 |
| Mineralöl | 5,00 |

(fortgesetzt)

| | Gew. -% |
|---|---|
| Hydriertes Polyisobuten | 5,00 |
| Vitamin E-Acetat | 0,50 |
| Octyltriazon | 2,00 |
| Butylmethoxydibenzoylmethan | 2,00 |
| Aerosil R972 | 0,50 |
| Aluminiumstearat | 1,00 |
| Konservierung | 0,50 |
| Glycerin | 10,00 |
| $MgSO_4$ | 1,00 |
| Wasser | ad 100 |

Die Caprylsäure/Caprinsäuretriglyceride, das Octyldodecanol, das Aluminiumstearat und das Butylmethoxydibenzoylmethan werden vereinigt und bei 120 °C verrührt, bis das Butylmethoxydibenzoylmethan bzw. das Aluminiumstearat aufgeschmolzen bzw. gelöst vorliegen. Diese Mischung wird auf 80 °C abgekühlt und mit den übrigen Komponenten der Fettphase vereinigt, welche daraufhin mit der Wasserphase verrührt werden. Nach Homogenisieren und Abkühlen auf Raumtemperatur wird eine W/O-Emulsion mit einem Gehalt an Al $[Dibenzo]_3$-Kügelchen erhalten.

**Beispiel 5**

W/O-Emulsion

| | Gew. -% |
|---|---|
| Cetyl Dimethicon Copolyol | 5,00 |
| Caprylsäure/Caprinsäuretriglyceride | 5,00 |
| Octyldodecanol | 5,00 |
| Dimethicone | 2,00 |
| Mineralöl | 5,00 |
| Isohexadekan | 5,00 |
| $C_{12-15}$ Alkylbenzoat | 5,00 |
| Octyltriazon | 4,00 |
| Methylbenzylidencampher | 4,00 |
| Butylmethoxydibenzoylmethan | 2,00 |
| Titandioxid | 2,00 |
| Aluminiumstearat | 0,50 |
| Konservierung | 0,50 |
| Glycerin | 5,00 |
| NaCl | 1,00 |
| Phenylbenzimidazolsulfonsäure | 4,00 |

(fortgesetzt)

| | Gew. -% |
|---|---|
| Natronlauge 45% | 1,30 |
| Wasser | ad 100,00 |

Die Caprylsäure/Caprinsäuretriglyceride, das Octyldodecanol, das Aluminiumstearat und das Butylmethoxydibenzoylmethan werden vereinigt und bei 120 °C verrührt, bis das Butylmethoxydibenzoylmethan bzw. das Aluminiumstearat aufgeschmolzen bzw. gelöst vorliegen. Diese Mischung wird auf 80 °C abgekühlt und mit den übrigen Komponenten der Fettphase vereinigt, welche daraufhin mit der Wasserphase verrührt werden. Nach Homogenisieren und Abkühlen auf Raumtemperatur wird eine W/O-Emulsion mit einem Gehalt an Al $[Dibenzo]_3$-Kügelchen erhalten.

**Beispiel 6**

Hydrodispersion

| | Gew.-% |
|---|---|
| Caprylsäure/Caprinsäuretriglyceride | 10,00 |
| Octyldodecanol | 5,00 |
| Dicaprylylether | 2,00 |
| Dimethicone | 1,00 |
| Vitamin E-Acetat | 0,50 |
| Octyltriazon | 2,00 |
| Methylbenzylidencampher | 4,00 |
| Butylmethoxydibenzoylmethan | 2,00 |
| Titandioxid | 1,00 |
| Aluminiumstearat | 2,00 |
| Konservierung | 0,50 |
| Glycerin | 3,00 |
| Xanthangummi | 0,40 |
| Pemulen TR1 | 0,40 |
| Natronlauge 45% | 0,40 |
| Wasser | ad 100,00 |

Die Caprylsäure/Caprinsäuretriglyceride, das Octyldodecanol, das Aluminiumstearat und das Butylmethoxydibenzoylmethan werden vereinigt und bei 120 °C verrührt, bis das Butylmethoxydibenzoylmethan bzw. das Aluminiumstearat aufgeschmolzen bzw. gelöst vorliegen. Diese Mischung wird auf 80 °C abgekühlt und mit den übrigen Komponenten der Fettphase vereinigt, welche daraufhin mit der Wasserphase verrührt werden. Nach Homogenisieren und Abkühlen auf Raumtemperatur wird eine Hydrodispersion mit einem Gehalt an Al $[Dibenzo]_3$-Kügelchen erhalten.

**Beispiel 7**

W/O-Pickering-Emulsion

| | Gew.-% |
|---|---|
| Caprylsäure/Caprinsäuretriglyceride | 15,00 |
| Octyldodecanol | 15,00 |
| Mineralöl | 5,00 |
| Hydriertes Polyisobuten | 5,00 |
| $C_{12-15}$ Alkylbenzoat | 5,00 |
| Octyltriazon | 1,00 |
| Methylbenzylidencampher | 2,00 |
| Butylmethoxydibenzoylmethan | 1,00 |
| Titandioxid | 4,00 |
| Aerosil R972 | 2,00 |
| Aluminiumstearat | 2,00 |
| Konservierung | 0,50 |
| Glycerin | 5,00 |
| NaCl | 1,00 |
| Phenylbenzimidazolsulfonsäure | 1,00 |
| Natronlauge 45% | 0,40 |
| Wasser | ad100,00 |

Die Caprylsäure/Caprinsäuretriglyceride, das Aluminiumstearat und das Butylmethoxydibenzoylmethan werden vereinigt und bei 120 °C verrührt, bis das Butylmethoxydibenzoylmethan bzw. das Aluminiumstearat aufgeschmolzen bzw. gelöst vorliegen. Diese Mischung wird auf 80 °C abgekühlt und mit den übrigen Komponenten der Fettphase vereinigt, welche daraufhin mit der Wasserphase verrührt werden. Nach Homogenisieren und Abkühlen auf Raumtemperatur wird eine W/O-Pickering-Emulsion mit einem Gehalt an Al $[Dibenzo]_3$-Kügelchen erhalten.

**Beispiel 8**

Ölgel

| | Gew.-% |
|---|---|
| Caprylsäure/Caprinsäuretriglyceride | 5 |
| Dicaprylylether | 5 |
| Dimethicone | 5 |

(fortgesetzt)

| | Gew.-% |
|---|---|
| Mineralöl | 30 |
| Isohexadekan | 10 |
| Hydriertes Polyisobuten | 20 |
| Butylen Glycol Dicaprylat/Caprat | 5 |
| $C_{12-15}$ Alkylbenzoat | 5 |
| Vitamin E-Acetat | 0,5 |
| Octyltriazon | 2 |
| Butylmethoxydibenzoylmethan | 1 |
| Aerosil R972 | 1 |
| Aluminiumstearat | 1 |
| Konservierung | 0,5 |

Die Caprylsäure/Caprinsäuretriglyceride, das Octyldodecanol, der Dicaprylylether, das Aluminiumstearat und das Butylmethoxydibenzoylmethan werden vereinigt und bei 120 °C verrührt, bis das Butylmethoxydibenzoylmethan bzw. das Aluminiumstearat aufgeschmolzen bzw. gelöst vorliegen. Diese Mischung wird auf 80 °C abgekühlt und mit den übrigen Komponenten der Fettphase vereinigt. Nach Abkühlen auf Raumtemperatur wird ein Ölgel mit einem Gehalt an Al $[Dibenzo]_3$-Kügelchen erhalten.

**Patentansprüche**

1. Lichtschutzwirksame Verbindungen des Aluminiums, welche das Strukturmotiv des Dibenzoylmethans enthalten.

2. Verbindungen nach Anspruch 1, dadurch gekennzeichnet, daß sie Komplexverbindungen des 4-(tert.-Butyl)-4'-methoxydibenzoylmethans und des Aluminiums darstellen.

3. Verbindungen nach Anspruch 1, dadurch gekennzeichnet, daß sie sich durch folgende Struktur bzw. eine tautomere Form dieser Struktur auszeichnen:

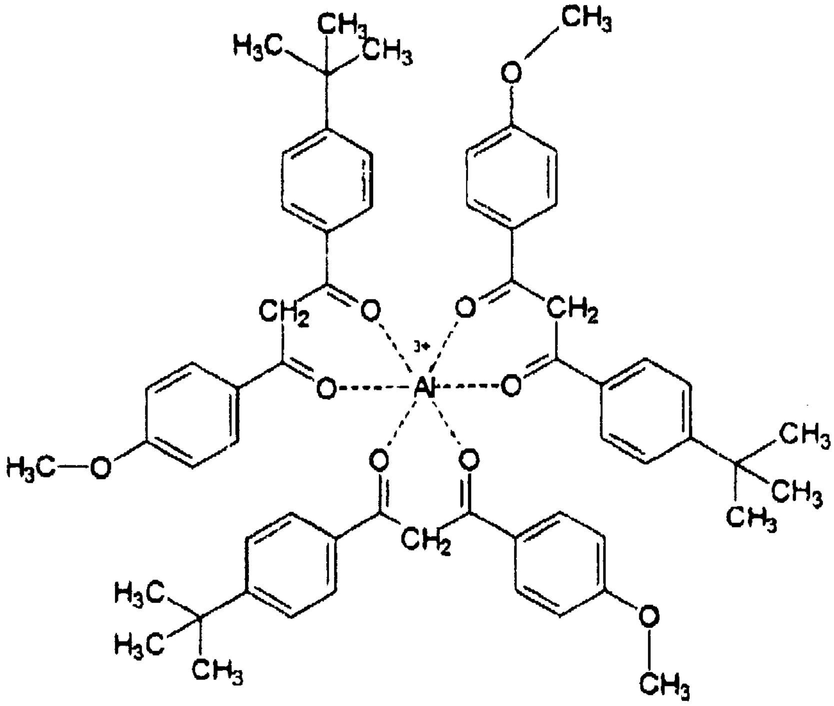

4. Kosmetische oder dermatologische Zubereitungeri mit einem Gehalt an einer Verbindung nach einem der Ansprüche 1 - 3.

5. Verwendung einer Verbindung nach einem der Ansprüche 1 - 3 zum Schutze der menschlichen Haut vor den schädiichen Einwirkung ultravioletter Strahlung.

6. Verfahren zur Herstellung von Verbindungen des Aluminiums, welche das Strukturmotiv des Dibenzoylmethans, dadurch gekennzeichnet, daß eine Lösung oder Suspension eines Dibenzoylmethanderivates, insbesondere des 4-(tert.-Butyl)-4'-methoxydibenzoylmethans, in einem geeigneten Lösungsmittel, vorteilhaft eines Öles, insbesondere einer kosmetischen Ölphase mit einem Aluminiumsalz, beispielsweise einem gelösten oder suspendierten Aluminiumsalz, insbesondere einem in einem Öl löslichen oder dispergierbaren Aluminiumsalz, vorteilhaft einem Aluminiumsalz einer Fettsäure, insbesondere vorteilhaft Aluminiumstearat versetzt wird.

7. Verfahren nach Anspruch 6, dadurch gekennzeichnet, daß bei der Vereinigung des Dibenzoylmethanderivates, insbesondere des 4-(tert.-Butyl)-4'-methoxydibenzoylmethans, mit dem Aluminiumsalz eine Temperatur im Bereich von ca. 85 °C - ca. 150 °C, insbesondere im Bereich von ca. 100 °C - ca. 140 °C, bevorzugt im Bereich von ca. 110 °C - ca. 130 °C gewählt und hernach auf Raumtemperatur abgekühlt wird.

Europäisches Patentamt

# EUROPÄISCHER RECHERCHENBERICHT

Nummer der Anmeldung
EP 99 12 0973

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 93 10753 A (RICHARDSON-VICKS) 10. Juni 1993 (1993-06-10) * Ansprüche 1-3,7 * | 1-5 | A61K7/42 |
| X | WO 93 11135 A (RICHARDSON-VICKS) 10. Juni 1993 (1993-06-10) * Anspruch 1; Beispiele 7-9 * | 1-6 | |
| X | EP 0 242 611 A (KAO) 28. Oktober 1987 (1987-10-28) * Ansprüche 1,2 * | 1,5 | |
| X | PATENT ABSTRACTS OF JAPAN vol. 14, no. 314, 5. Juli 1990 (1990-07-05) & JP 02 108614 A (SANSHO SEIYAKU), 20. April 1990 (1990-04-20) * Zusammenfassung * | 1,5 | |

RECHERCHIERTE SACHGEBIETE (Int.Cl.7)
A61K

Der vorliegende Recherchenbericht wurde für alle Patentansprüche erstellt

| Recherchenort | Abschlußdatum der Recherche | Prüfer |
|---|---|---|
| DEN HAAG | 20. März 2000 | Voyiazoglou, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 99 12 0973

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-03-2000

| Im Recherchenbericht angeführtes Patentdokument | Datum der Veröffentlichung | Mitglied(er) der Patentfamilie | Datum der Veröffentlichung |
|---|---|---|---|
| WO 9310753 A | 10-06-1993 | AU 3132193 A | 28-06-1993 |
| | | CN 1074909 A | 04-08-1993 |
| | | MX 9206875 A | 01-06-1993 |
| | | PT 101102 A | 28-02-1994 |
| WO 9311135 A | 10-06-1993 | AU 3072192 A | 28-06-1993 |
| | | CN 1073858 A | 07-07-1993 |
| | | MX 9206876 A | 01-06-1993 |
| | | PT 101103 A | 28-02-1994 |
| EP 242611 A | 28-10-1987 | JP 1010492 B | 22-02-1989 |
| | | JP 1525576 C | 30-10-1989 |
| | | JP 62240611 A | 21-10-1987 |
| | | DE 3774160 A | 05-12-1991 |
| | | KR 9507906 B | 21-07-1995 |
| | | US 4837010 A | 06-06-1989 |
| JP 02108614 A | 20-04-1990 | JP 2965076 B | 18-10-1999 |

EPO FORM P0461

Für nähere Einzelheiten zu diesem Anhang : siehe Amtsblatt des Europäischen Patentamts, Nr.12/82